Europäisches Patentamt

European Patent Office

Office européen des brevets

(19)

(11) Publication number: **0 498 888 A1**

## EUROPEAN PATENT APPLICATION
## published in accordance with Art. 158(3) EPC

(12)

(21) Application number: 90916053.3

(22) Date of filing: **05.11.90**

(51) Int. Cl.5: **G01N 27/06**

(86) International application number:
**PCT/JP90/01434**

(87) International publication number:
**WO 91/06848 (16.05.91 91/11)**

(30) Priority: **04.11.89 JP 286025/89**

(43) Date of publication of application:
**19.08.92 Bulletin 92/34**

(84) Designated Contracting States:
**CH DE FR GB LI**

(71) Applicant: **NIHON MILLIPORE KOGYO KABUSHIKI KAISHA**
**4736-3, Hachimanpara 2-chome**
**Yonezawa-shi Yamagata 992-11(JP)**

(72) Inventor: **HIROSE, Atsumi**
**1735-32, Kamikurata-cho Totsuka-ku**
**Yokohama-shi Kanagawa 244(JP)**
Inventor: **KANAZAWA, Masanori**
**1018-103, Kamiochiai**
**Yono-shi Saitama 338(JP)**

(74) Representative: **Henkel, Feiler, Hänzel & Partner**
**Möhlstrasse 37**
**W-8000 München 80(DE)**

(54) METHOD OF MEASURING TOTAL OUANTITY OF ORGANIC SUBSTANCES IN ULTRAPURE WATER AND ULTRAPURE WATER TREATING SYSTEM UTILIZING SAID METHOD IN PREPARATION OF ULTRAPURE WATER.

(57) This invention relates to a method of measuring the total quantity of organic substances in ultrapure water and an ultrapure water treating system utilizing said method, and also provides a method capable of simple and accurate on-line measurement of TOC quantity in ultrapure water with one set of sensor and an on-line ultrapure water treating system capable of stably supplying ultrapure water containing an extremely small quantity of TOC. The method comprises applying ultraviolet rays to ultrapure water whose specific resistance value has been set to a known constant one by ion exchange treatment, finding decreases by subtracting specific resistance values of ultrapure water measured continuously at different points from the abovesaid constant specific resistance value, and measuring a quantity of TOC on the basis of correlation of the decreases with the quantity of generated $CO_2$ and so on converted into the total content of organic substances in ultrapure water. The system is composed on ion exchange means (13); ultraviolet treating means (14) for decomposing organic substances by exposing ultrapure water, which is supplied from the ion exchange means (13); to ultraviolet rays; means (15) for measuring a specific resistance value of ultrapure water subjected to the above treatment; ion exchange means (16) for restoring a specific resistance value above a predetermined one; a valve (17) capable of changing over the flow direction; a reflux pipe (18) to form a circulation passage connected between one of the outlets of the valve (17) and the upstream side of said ion exchange means (13); and monitor means (20) for finding a total content of organic carbon in ultrapure water on the basis of the correlation of a TOC quantity in ultrapure water with the difference between a measured specific resistance value of ultrapure water and a pre-fixed constant one.

EP 0 498 888 A1

# Fig. 3

# Fig. 4

FIELD OF THE INVENTION

The present invention relates to a method of measuring the total amount of organic substances in ultrapure water in the production of ultrapure water and an ultrapure water treatment system employing the method. Particularly, the present invention relates to a method of measuring the amount of organic substances in ultrapure water when the organic substances are reduced to trace amounts by the ultraviolet decomposition of the organic substances, and also to an ultrapure water treatment system, using such method, by which is produced a low-organic-substance-containing ultrapure water having not more than a predetermined value of organic substance content therein.

BACKGROUND ART

Ultrapure water is a purified water, from which impurities are removed at the greatest possible amount, having a resistivity value of 16 to 18 M$\Omega$ -cm. Ultrapure water at present is widely used as a washing water in production of semiconductors, medical raw materials, chemical raw materials and the like.

The quality of such ultrapure water is strictly controlled with respect to four items, i.e., resistivity, organic substances, fine particles and microorganisms. In particular, ultrapure water used for VLSI is significantly strictly controlled with respect to the amount of total organic substances (Total Organic Carbon: referred to as TOC hereinafter), in contrast to other ultrapure water in other applications. The reason is that, when the organic substances in ultrapure water adhere to and remain on the surface of a wafer, oxidation speed in oxidizing the wafer surface may vary at different locations on the wafer surface, causing such quality failure as a pinhole and the like and thus reducing the yield of resultant wafer extraordinarily. In addition, with the improved resolution found in analytical instruments and apparatus in the fields of pharmaceutical and organic chemical research, bioengineering, other high-precision experiments and the like, a great concern is directed to a reduction of organic substance amount in ultrapure water, to achieve a higher grade of analytical technique.

In recent years, there are therefore demands for measurement of the trace organic substances remaining in ultrapure water and reduction in the amounts thereof to 10 ppb or less.

Since many kinds of organic substances dissolve in water, it is hardly possible to quantitatively determine each of the organic substances. The analysis of water thus employs a method of quantitatively determining carbon in the constituent elements of organic substances, which is used as an index indicating a total amount of organic substances. This organic carbon quantitation involves measuring $CO_2$ obtained from an organic carbon decomposed by oxidization treatment, because carbon (C) in an organic polymer can not directly be measured.

An example of conventional methods and apparatus for measuring the organic carbon content of ultrapure water is the Regan patent (U.S.Patent No. 3,958,941). The Regan patent employs a two-loop system in which the organic substances in water are first oxidized by exposing them to ultraviolet light, and the carbon dioxide produced is then transferred to a separate measurement room in which the carbon dioxide is dissolved in pure water, and the conductivity of the resultant solution is then measured. Therefore, the conductivity is measured in such room separate from the location where the ultraviolet light is applied to water. This situation obviously requires transferring the carbon dioxide between the ultraviolet light irradiation room and conductivity measuring room. This is a significant fault. Although the present invention has been achieved with a view to measuring a low organic substance content in ultrapure water, mixing of any small impurities, which is caused by transfer or the like, is an important factor which hinders precise measurement, like the loss of carbon dioxide.

In addition, the Regan apparatus is based on the premise that the time required for completing the oxidation process is constant. Hence, if the organic substances present in a sample are hardly oxidized, or if irradiation is insufficiently effected for oxidation within a predetermined time due to a deterioration in a lamp, the measurement will be made in error and values obtained will be lower. In addition, if oxidation is rapidly completed due to a low concentration of organic substances, there is the possibility that significant error is created by the interference of the error caused by the instrument used. Consequently, the Regan patent should be used mainly in a region of relatively high concentrations of organic substances.

Japanese Patent Publication No. 63-46375 (U.S.Patent No. 4,666,860) is therefore proposed for improving the above-described faults. In the measurement method disclosed in Japanese Patent Publication No. 63-46375, the inside of a single cell to be used is divided by a quartz window into a sample chamber provided with means for measuring the conductivity of ultrapure water and a chamber provided with an ultraviolet-light irradiation lamp. In this manner, a predetermined amount of ultrapure water sample is introduced into the sample chamber provided with the measuring means and oxidized by irradiation with ultraviolet light, and a change in conductivity of the ultrapure water, which is caused by $CO_2$, is measured in the same sample chamber. In this apparatus for measuring organic carbon content, the ultrapure water sample, which is sampled at predetermined intervals from the same process, is decomposed in a rested state by irradiation with ultraviolet light in the sample chamber, and the conductivity is then measured, like the Regan patent. However, during the time the ultrapure water stands still for oxidation and measurement, soluble substances are dissolved, which affect the conductivity of ultrapure water and the amount of such soluble substances increases significantly such that it cannot be disregarded. As a result, a complicated precise calibration means and calibration process are required, and a process is also required to assure that oxidation is completed. These requirements have an influence on the measurement precision and make the apparatus complicated and expensive. Further, the most important problem is that the foregoing conventional measurement methods and apparatus are adapted for use on water samples which are sampled (i.e. batch measurement), and thus they are not applicable to on-line-type measurement methods and apparatus which continuously monitor a bulk flow of ultrapure water in a stationary state, as in an ultrapure water production system which the present invention concerns.

On the other hand, a technique which employs the two sample cells disclosed in the above-described Japanese Patent Publication No. 63-46375 is proposed as an apparatus for monitoring changes in the organic carbon content in ultrapure water which constantly flows. In this system, the two sample cells for measuring the conductivity of ultrapure water are connected to each other in series relative to the flow of the ultrapure water sample, at least the sample cell on the downstream side being provided with a window made of a material which is substantially transparent for ultraviolet light, and further provided with an ultraviolet light source which is disposed at a side opposed to the window. In this prior art, the sample cell on the upstream side operates without the ultraviolet light irradiation, at which step, the conductivity measured in this upstream-side cell is indicative of an ionic conductivity, or the background value, in the flowing stream of ultrapure water sample. A difference between this ionic conductivity and the conductivity measured in a sample cell on the downstream side is monitored for deciding as to whether or not the organic carbon content in the flow of the ultrapure water changes.

However, because this conventional method of measuring organic substances requires two sensors for measuring the conductivity of ultrapure water at the respective positions before and after the ultraviolet-light treatment means, it is a problem that the apparatus used is complicated in structure, and the maintenance is thus troublesome. In addition, an electrode in the upstream-side cell, by reason of its not being irradiated with ultraviolet light, becomes fouled with time, and an error is thus easily produced in the measurement of the background value. Such error causes an inaccuracy, leaving undesired obstacles in measuring such an extremely small quantity of TOC which is of a major concern in the ultrapure water system. Further, according to the prior art, the conductivity is monitored at a same point while simultaneously oxidizing organic matters by the ultraviolet-light irradiation, which makes the oxidation incomplete or varies the oxidation rate at local points, thereby resulting in significant dispersions in the measurement values. Consequently, as pointed out in the above-described Japanese publication, the method set forth above is not sufficient to reliably measure an absolute value for the amount of organic matters, and therefore its possible use in this context is confined to a cautionary measure for indicating a rapid change in the amount of organic matters.

DISCLOSURE OF THE INVENTION

It is an object of the present invention to provide an on-line type measurement method which is capable of continuously and accurately measuring, by use of simplified devices, the amount of total organic matters in an ultrapure water which flows at a constant flow rate in an ultrapure water production system. It is another object of the present invention to provide an on-line type ultrapure water treatment system which is capable of stably producing an ultrapure water having a low organic matter content.

In order to achieve those purposes, according to the present invention, there is provided a method of measuring an amount of total organic matter content in an ultrapure water in an ultrapure water production system in which substantially all of said first ultrapure water being flowed is exposed to an ultraviolet light for decomposing the organic matter therein, to thereby produce a second ultrapure water having a low organic matter content. The invention is further characterized in that an ultraviolet treatment is performed to the first ultrapure water which has undergone an ion-exchange/organic-matter-removing treatment, the first ultrapure water having a predetermined resistivity value or conductivity. A measurement is continuously made of the first ultrapure water immediately after the exposure to said ultraviolet light: thereafter a measurement value obtained by such measurement is subtracted from a known predetermined resistivity value or conductivity to determine a resistivity difference. From that resistivity difference, by referring to a correlation between a resistivity or conductivity difference and an amount of carbon dioxide or the like produced in ultrapure water, the amount of total organic matter content in the second ultrapure water is determined.

In another aspect of the invention, there is provided an ultrapure water system for obtaining an ultrapure water having a predetermined resistivity value or conductivity from a primary pure water source that has undergone a preliminary treatment by ion exchange, reverse osmosis, distillation or the like characterized in that the ultrapure water system comprises an ion-exchange/organic-matter-removing means. An ultraviolet treatment means is provided for exposing the ultrapure water being supplied from such ion-exchange/organic-matter-removing means to an ultraviolet light, thereby decomposing organic matters therein. A means is also provided for measuring a resistivity value or conductivity of the ultrapure water being introduced immediately after the exposure to the ultraviolet light, together with an ion exchange means by which any resistivity value that is varied due to ions generated from the organic matter decomposed by the ultraviolet irradiation is brought back to a predetermined value or higher. Also provided is a valve able to change over a direction in which the ultrapure water is flowed, a reflux pipe which connects one of the discharge ports of the valve with an upstream side of the ion-exchange/organic-matter-removing means to form a circulation passage, and a monitor means. The monitor means determines a difference between the resistivity value or conductivity of the ultrapure water that has undergone the ultraviolet treatment and been measured by the resistivity value or conductivity measuring means and a known predetermined resistivity value or conductivity of ultrapure water prior to the ultra-violet treatment. The monitor means also determines the amount of total organic matter content in the ultrapure water on the basis of a correlation between the resistivity or conductivity difference and an amount of $CO_2$ produced or such substance as an organic acid that can be ionized in the ultrapure water.

In the foregoing ultrapure water system, a resistivity/conductivity measuring means may be used for quality control on a downstream side of the ion exchange means in order to detect whether or not the resistivity value of ultrapure water is brought to a predetermined value.

Further, in the ultrapure water system, a resistivity/conductivity measuring means for quality control may be interposed between the ion-exchange/organic-matter-removing means and the ultraviolet treatment means in order to detect whether or not the resistivity value of ultrapure water is brought to a predetermined value.

Therefore, according to the present invention, an ultrapure water, whose resistivity (or conductivity) has been set as a known predetermined value, e.g. 18 MΩ -cm by the ion-exchange/organic-matter-removing means, is supplied to the ultraviolet treatment means. In this respect, as the ultrapure water is being flowed at a constant rate, no change of water resistivity (or conductivity) occurs substantially due to substances eluted from the material surrounding the water. Thus, as far as the ultrapure water obtained shortly after the exposure to the ultraviolet light is concerned, a reduction of resistivity value in that water may be determined, depending on the amount of $CO_2$ or such substance as organic acids that can be ionized, which is generated in decomposing organic matters in the ultrapure water by means of ultraviolet light. Further, as the water flows at a constant rate within the ultraviolet treatment means, the ultraviolet light is applied thereto at a constant amount, whereupon it follows that the amount of organic matter content in the ultrapure water can be determined from the $CO_2$, organic acid products or the like generated therein.

According to the invention, therefore, a measurement of resistivity is continuously made on the ultrapure water flowing immediately after the exposure to ultraviolet light. The measurement value thus obtained is subtracted from a known resistivity value of a pre-ultraviolet-treatment ultrapure water that is to undergo the ultraviolet treatment; that is, a predetermined resistivity value as required in the ultrapure water production, so as to obtain a resistivity difference between the measured value and the predetermined value. From this result, as well as on the basis of a correlation between the reduction of resistivity after the ultraviolet treatment and $CO_2$ or such substance as organic acids that can be ionized in the ultrapure water, an estimation or prediction may be made as to the amount of total organic matter content in the ultrapure water. The amount thus estimated may be indicated as a TOC amount on a display device and be transmitted as a signal to a passage change-over valve to control the direction of flow of ultrapure water. When the TOC amount is determined to be greater than a predetermined value, the ultrapure water can be re-circulated through the ion-exchange/organic-matter-removing and ultraviolet treatments, so that any residual organic matters are decomposed and removed in the water, to thereby supply an ultrapure water having a TOC content amount which is constantly kept to the smallest possible quantity.

BRIEF DESCRIPTION OF THE DRAWINGS

Fig. 1 is a block diagram showing a general ultrapure water production system;

Fig. 2 is a block diagram of an ultrapure water treatment system which is the same production system in accordance with one embodiment of the present invention;

Fig. 3 is a block diagram of an ultrapure water treatment in accordance with another embodiment of this invention;

Fig. 4 is a block diagram of an ultrapure water treatment system in accordance with a still another embodiment of this invention;

Fig. 5 is a longitudinally sectional view taken along the center of an electrode of resistivity or conductivity measurement means;

Fig. 6 is a sectional view taken along the line VI-VI of the electrode shown in Fig. 5; and

Fig. 7 is a graph showing a relation between the amount of total organic matter and reduction in resistivity value after ultraviolet-light treatment of ultrapure water, which is experimentally obtained by the ultrapure water treatment system shown in Fig. 2.

BEST MODE FOR CARRYING OUT THE INVENTION

The present invention will be described in detail below with reference to the embodiments shown in the drawings.

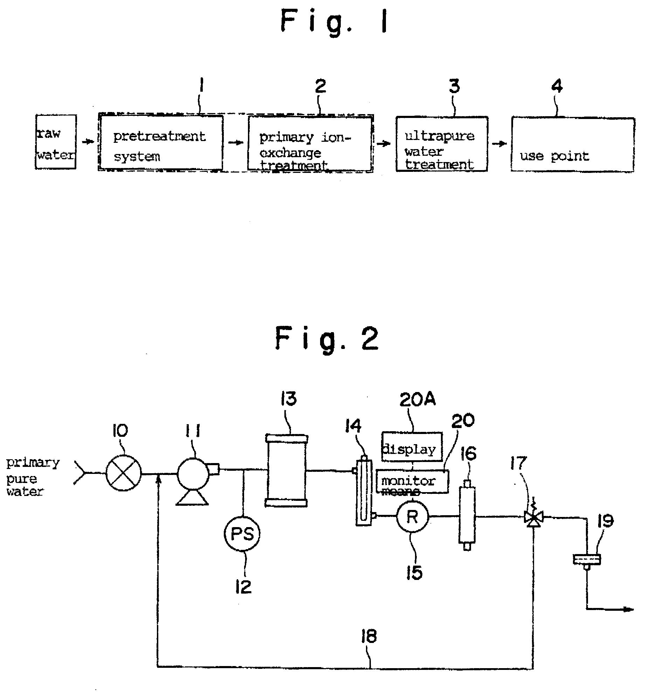

An ultrapure water system is generally roughly divided into a primary pure water system and a sub-system. In the primary pure water system, a raw water, such as industrial water, city water or the like, is treated into a pure water having resistivity of 7 to 14 MΩ -cm, while removing most of fine particles and organic substances therefrom. Principle components of such a pure water system generally include a filter apparatus, a reverse osmosis membrane apparatus, a deaerator and an ion exchanger. Practically, those components are appropriately selected and used separately or in combination as may be required. According to an ultrapure water production system in the present embodiment, as shown in Fig. 1, its primary pure water system comprises a pretreatment system 1 and a primary ion exchange treatment system 2, with an ultrapure water treatment system 3 incorporated as a sub-system.

The pretreatment system 1 is provided for removing impurities from the raw water, such as city water, recycled drainage or the like, so that this water can be converted to a primary pure water at a subsequent primary ion-exchange stage. For example, the pretreatment system 1, comprises a combination of a prefilter, a reverse osmosis membrane apparatus and other required apparatus such as a filter system, a deaerator and the like. Typically, the pretreatment system 1 consists in firstly using the prefilter to remove part of organic substances and large impurities, i.e. large particles and colloidal substances, in raw water, so as to prevent blinding or damage of the reverse osmosis membrane on the downstream side. The prefilter is generally a depth filter or the like. Secondly, the reverse osmosis membrane apparatus, using a reverse osmosis process, separates and removes most other contaminants in raw water, such as inorganic ions, oragnic substances, pyrogens, colloid and fine particles of 1 mm or more, microorganisms and the like, to produce pure water. The reverse osmosis membrane may generally include a semipermeable membrane, such as a cellulose acetate membrane, a polyamide membrane, and polysulfonate membrane. It is noted that the raw water is supplied to this reverse osmosis membrane, after having passed through the prefilter, via a booster pump.

The primary ion-exchange treatment system 2 uses an ion-exchange membrane to remove dissolved inorganic ions or other organic matter from the foregoing raw water that has undergone the reverse osmosis treatment to obtain a primary pure water whose resistivity is raised to approximately 7-14 MΩ -cm. The ion-exchange membrane for common use in this system is a mix bed type of membrane having a mixture of cation and anion exchange resins therein.

After the foregoing primary treatment, the primary pure water (resistivity: 7~14 MΩ -cm) is introduced into the ultrapure water treatment system 3 and purified therein to produce ultrapure water of 17 MΩ -cm or more, preferably 18 MΩ -cm or more. As shown in Fig. 2, the ultrapure water treatment system 3 comprises; an integrating flowmeter 10 for measuring the amount of the primary pure water supplied to the ultrapure water treatment system 3; a pump 11 for sending, under pressure, the primary pure water to an ion exchange/organic substance removing means 13; a pressure switch 12 which is sensitive to a change in the pressure of primary pure water within the tubings so as to inactivate pump 11. This is effective, for example, when a change occurs due to the blinding of the above-mentioned means 13, or discontinuation of the supply of the primary pure water. The ion exchange/organic-matter-removing means 13 is provided to adsorb inorganic ions and the like in the primary pure water so as to increase the resistivity value to obtain an ultrapure water having such a predetermined resistivity value as 18 MΩ -cm. An ultraviolet treatment means 14 is provided in which the ultrapure water being supplied from the means 13 is exposed to ultraviolet light for decomposing organic matters, and a means 15 is also provided for measuring the resistivity value of such ultrapure water flowing immediately after exposure to the ultraviolet light. A monitor means 20 determines the difference between the resistivity value or conductivity of the ultrapure water which is measured by the resistivity value or conductivity measuring means after the ultraviolet treatment and a known constant resistivity value or conductivity of ultrapure water set before the ultraviolet treatment. This difference provides an estimate of the total organic carbon content in the ultrapure water on the basis of a correlation between such resistivity difference or conductivity difference and carbon dioxide produced in the ultrapure water. An ion exchange means 16 is included for removing ions resulting from oxidation of organic matter exposed to ultraviolet light to again increase the resistivity value of the ultrapure water to a predetermined value, for example, 18 MΩ -cm or more. A passage change-over valve 17 is operable to change the flow direction of treated water, and a reflux pipe 18 connects one of the discharge ports of the passage change-over valve 17 with the upstream side of the ion exchange/organic substance removing means 13, for example, a point upstream of the pump 11, thereby defining a flow circulation passage. A final filter 19 is also provided comprising a membrane filter or the like. The ultrapure treatment system 3 causes an ultrapure water to flow at a constant flow rate under control of the pump 11.

The ion exchange/organic substance-removing means 13 performs a final treatment for removing ions remaining in the primary pure water so as to bring the resistivity value of ultrapure water to a predetermined value, for example, 18 MΩ -cm. Such means 13 is generally an ion exchange device containing a mixture of cation exchange and anion exchange resins, each being refined to high purity, but the means 13 is not limited thereto. A housing of this ion exchange/organic substance-removing means 13 is formed from a material not prone to release any substances affecting the resistivity value of ultrapure water, preferably, polypropylene or the like. The ion exchange resin for use in the means 13 comprises highly refined cation and anion exchange resins having extremely limited elutes which would adversely affect the TOC in the ultrapure water. An activated carbon can be mixed with those two ion exchange resins.

At the ultraviolet treatment means 14, organic substances, such as lower alcohols, ketones and the like, which are hard to remove by the reverse osmosis membrane apparatus in the primary pure water treatment system, can be removed by means of ultraviolet light which effects oxidation. The means 14 exposes those organic substances to ultraviolet light in order to decompose them into carbon dioxide or organic acid ions. In regard to TOC present in very small quantities in ultrapure water, the inter-molecular bonds of organic substances are cut by hydroxyl radicals having an oxidation potential end the energy of ultraviolet light so that the organic substances are decomposed into organic acids and finally carbon dioxide and water. In this respect, the irradiation with ultraviolet light suffices to oxidize the organic substances without adding a particular oxidizing agent because the ultrapure water contains sufficient oxygen. The ultraviolet treatment means 14 may be a known ultraviolet oxidation apparatus, but preferably be one employing a low-pressure mercury lamp which emits ultraviolet light of approximately 185 nm wave length which is effective in decomposing organic compounds. This is however not limitative. Typically, a low-pressure mercury lamp emits only about 3% of the 185 nm ultraviolet light, and most of the remaining emitted ultraviolet light is of 253.7 nm wave length with a high bactericidal effect. Thus, a glass allowing those two wavelengths of ultraviolet light to be transmitted therethrough should be used as a partition glass in the ultraviolet oxidation device. The partition glass defines an ultraviolet lamp compartment in which the relevant UV lamp is contained and a water passage compartment through which the ultrapure water passes. The partition glass is preferably a "Supersil" glass produced by Ameresil Co.. The volume surrounding the ultraviolet lamp in the light-source compartment divided by the above-mentioned glass contains a dry $N_2$ or a gas which does not absorb an ultraviolet light of approximately 185 nm wave length. The organic substances to be removed in this ultrapure water system are believed to have relatively lower molecular weights that have not removed by the primary pure water system. In decomposing such organic systances, it is founed that the rate with which the organic substances are decomposed correlates with the amount of ultraviolet light applied thereto, that is, the TOC in the ultrapure water is infinitely decreased with an increase of ultraviolet light irradiation amount. From this viewpoint, it is essential that the amount of ultrapure water introduced into the ultraviolet treatment means 14, or strictly stated, the amount of ultrapure water being flowed in the present ultrapure water system, should be kept constant, while further positively securing the foregoing ultraviolet light energy quantity. To meet that requirement it is desirable to adjust the pump 11 and flow meter 10 for proper control.

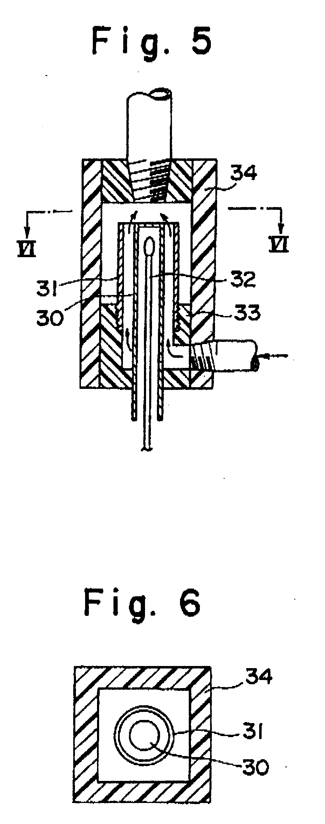

The resistivity meansuring means 15 may comprise such a known resistivity meter as available e.g. from Millipore France Co. (Model ZF-2000011), which is adapted for use in ultrapure water and performs continuous measurement on changes in resistivity value and conductivity. Electrodes used in the means 15 may be the one shown in Figs. 5 and 6 as a preferred embodiment. The construction of this electrode is such that there are provided a cylindrical electrode 30 having a closed one end and another cylindrical electrode 31 having both ends opened completely, both of these two electrodes being concentrically disposed on an insulating block 30. This allows the whole ultrapure water discharged from the ultraviolet treatment means 14 to flow between the two electrodes. This concentric arrangement of electrodes 30, 31 contributes to an extremely high increase of cell constant, thus improving the relative sensitivity of the meter to changes in conductivity irrespective of these electrodes being relatively smaller in electrostatic capacity between them. A thermistor as a temperature sensor 32 is incorporated in the inner electrode 30 and is set to compensate for changes of resistivity value or conductivity resulted from temperature changes of ultrapure water, and to convert the resistivity value as if it is measured at a constant temperature, such as at 25°C by the electrodes 30, 31. Although, in this embodiment, stainless steel (SUS316) is used as a material for the electrodes 30, 31, the material is not limited to this. Other suitable materials include titanium, palladium, iridium, rhodium, platinum or the like. A voltage applied to the electrodes, for example, may be constant within the ranges of 10 mV to 100 mV. The resistivity meter used may have approximately 0.1 to 0.01 MΩ -cm sensitivity (resolution), preferably 0.01 MΩ -cm. The output from the electrodes 30, 31 is input via an A/D converter into the monitor means 20 comprising a computer or a data processing device equivalent thereto so that the resistivity or conductivity of the ultrapure water passed through cell is monitored over time.

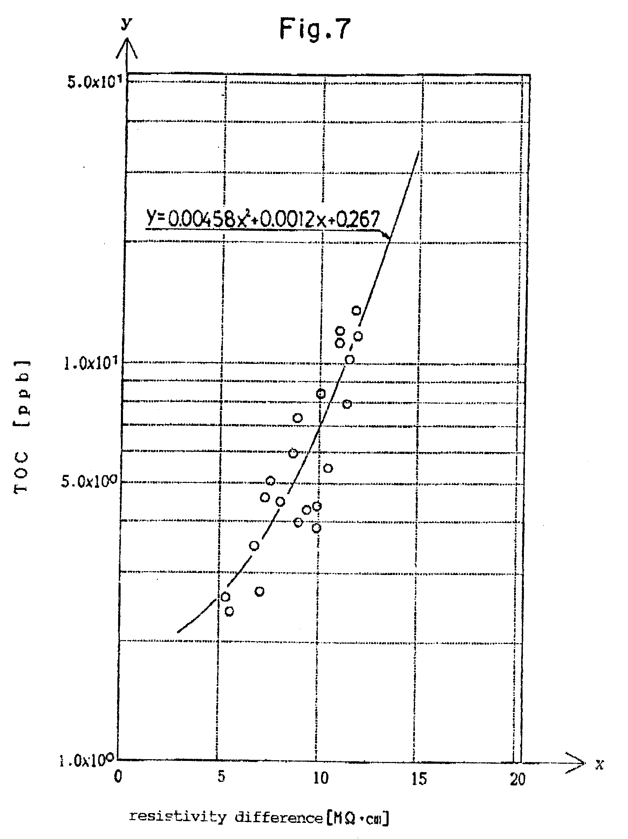

The monitor means 20 functions to continuously measure the resistivity value (or conductivity) of ultrapure water being introduced shortly after the exposure to ultraviolet light, and then determine a resistivity difference $x$ by subtracting the measured resistivity value from a given resistivity value of ultrapure water or a known resistivity value (or conductivity), 18 MΩ -cm, as generally characterizes ultrapure water. Further, the monitor means 20 operates to make reference to a correlation between the resistivity difference $x$ , which is thus determined from the ultrapure water before and after the ultraviolet decomposition treatment, and the amount of $CO_2$ produced in the ultrapure water or that of such anions as organic acid ions and the like produced in the same, and then estimates the total amount of organic matters in the water according to that correlation. That correlation between the conductivity of ultrapure water and carbon dioxide content therein has been known. For example, the correlation is described in "A New Approach to the Measurement of Organic Carbon", Poirier et al, American Laboratory, Dec. 1978, and reference may be made to this generally known correlation, but a personal experiment may be conducted to measure a practical resistivity difference $x$ in ultrapure water before and after the ultraviolet treatment and a practical amount of $CO_2$ produced or that of oxidized TOC so as to establish an original correlation therebetween. A reduction of resistivity in the ultrapure water being flowed is measured to attain the resistivity difference $x$ , and by applying this resistivity difference to either of the foregoing known and personal correlations, there may be obtained TOC in that flowing ultrapure water. The TOC value thus determined may be indicated on a display 20A and transmitted as a control signal to the passage change-over valve 17 in order to automatically change the ultrapure water flow to a use point. As far as the personal experiment is concerned, the ultrapure water, which is obtained by the ion exchange treatment, bringing its resistivity to a known value (18 MΩ -cm) constantly, is measured for the reduction of its resistivity subsequent to the ultraviolet decomposition treatment, and then the resistivity reduction is compared to a value of oxidized TOC amount that was obtained by the experiment in the ultrapure water, having a given correlation with the resistivity fall degree, whereby the estimation or prediction is made from the experimental value. This kind of estimation may be effected by a processing and inference means comprising a known computer and programs for driving the computer. As stated earlier, an original correlation may be obtained experimentally between the produced $CO_2$ or organic acids, which is converted into a TOC, and the resistivity reduction in the water after the ultraviolet decomposition treatment. An example of this original correlation is shown in Fig. 7. The correlation is statistically expressed by such an operation expression as the following equation (1):

$$y = 0.00458x^2 + 0.0012x + 0.267 \qquad (1)$$

The total content y of organic carbon in ultrapure water is calculated by the above correlation expression. In operation, the amount of organic matters in the ultraviolet water is determined by obtaining the y value in using the foregoing equation, and when the organic matter amount reaches a desired value, the three-way value 17 is switched so as to cause the ultrapure water to flow into the use point 4. The operation expression showing this correlation is not limited to the Equation (1), and it varries depending upon the intensity of ultraviolet light and the components of organic substances. It is therefore preferable to provide a certain width of error range, i.e., an allowable range, for the conditions shown in Fig. 7.

The ion exchange means 16 comprises an anion exchange resin for anion exchange and functions to remove the ion produced by the ultraviolet decomposition apparatus. The ion exchange means 16 has a housing of a material which contains extremely small quantities of solute affecting the TOC amount of ultrapure water; for example, polypropylene, which is economical and inexpensive. It is important that the anion exchange resin completely adsorbs and removes the anions and moieties of the organic substances that have been produced at the ultraviolet treatment means 14. For this ion exchange means 16, a mixed bed ion exchange device may be employed, which comprises anion and cation exchange resins therein.

The final filter 19 is an ultrafilter which is adapted to remove fine particles, some of which have escaped from the primary pure water system and another of which have been admitted into or generated in the water at the terminal tubings within the ultrapure water system. This ultrafilter also serves to completely remove bacteria so as to prevent the terminal tubings from bacterial contamination. The ultrafiltration element used for the final filter 19 is not limited to any particular construction, but the preferable one may be an external-pressure-type ultrafiltration element using hollow fibers.

In Fig. 2, reference numeral 20A denotes a known display for displaying the total amount of organic substances in ultrapure water. It is desirable that at least water contact points in the respective devices or elements forming the ultrapure water system, which are in contact with the ultrapure water, should be formed with inert materials hard to react with the ultrapure water to prevent elution of additional impurities that might change the resistivity or conductivity of the ultrapure water.

The primary pure water, once admitted into the ultrapure water system, is treated at the ion-exchange/organic-matter-removing means 13 so that the residual inorganic ions and organic matter in the primary pure water are removed therefrom to obtain a predetermined ultrapure water, such as the water of 18 $M\Omega$ -cm resistivity. The whole of ultrapure water thus produced is then supplied to the ultraviolet treatment means 14 for oxidization of organic matter therein to reduce the TOC value. As is well known, constantly flowing ultrapure water is least affected in a change conductivity by materials eluted from the devices and elements of the system, such as the tubings or the ultraviolet treatment apparatus 14. This condition applies to a stream of ultrapure water obtained immediately after treatment by the ion-exchange membrane, with the resistivity of the water adjusted to a given value: Namely, the state of ultrapure water can be determined positively to have such a constant resistivity value of 18 $M\Omega$ -cm or more. Accordingly, one can assume that the ultrapure water being supplied from the ion-exchange/organic-matter-removing means 13 has a known and constant resistivity value. The exposure of featured ultrapure water to the ultraviolet light in the ultraviolet treatment means 14, effects oxidation of the extremely small quantities of organic matter that can not be removed through the previous treatments. The organic matter is decomposed into water and carbon dioxide gas ($CO_2$), with the formation of carbonate ions ($CO_3^{++}$) particularly by ultraviolet light of approximately 185 nm wavelength. The generation of carbonate ions in this organic matter decomposition stage causes a sudden reduction in the resistivity of ultrapure water flowing immediately after exposure to the ultraviolet light. This resistivity reduction can be determined from the amount of $CO_2$ being produced or that of other substances which can be ionized, such as organic acids. In the ultraviolet treatment means 14, the water is flowed at a constant rate, so that the water is exposed to a constant amount of ultraviolet light. Under this operating condition, the above-stated resistivity reduction is related to an organic carbon content in the ultrapure water, even though the oxidation in the ultraviolet treatment device may not be completed during a short exposure time.

It is not necessary to monitor the entirety of the oxidation process in order to secure a precise result, when monitoring the total organic carbon content in the stationary stream of ultrapure water. Therefore, in accordance with the present invention, measurement of resistivity is continuously made on the ultrapure water flowing shortly after exposure to the ultraviolet light by use of the resistivity and conductivity measuring means 15 to monitor the reduction of resistivity us stated above. In other words, a calculation is made by subtracting the values of resistivity measured shortly after such ultraviolet treatment from a given ultrapure water resistivity value (such as 18 $M\Omega$ -cm) set prior to the ultraviolet treatment, so as to obtain the resistivity difference $x$ . Then, referring to the known or original correlation between the ultrapure water resistivity (or conductivity) and carbon dioxide production, the calculation is made on the basis of that correlation to determine a total organic carbon content in the ultrapure water. In operation, the resistivity values measured are subjected to the subtraction operation at the monitor means 20 which comprises, for example, a differential amplifier circuit and a processing unit, such as a known computer or the like. The subtraction is made in comparison with a reference resistivity or the established preset resistivity value (18 $M\Omega$ -cm) of ultrapure water introduced in the ultraviolet treatment device 14, to thereby obtain the resistivity difference. Next, the correlation between the resisitivity difference and TOC is established through a comparative computation, using a comparator circuit and a processing unit such as a known computer or the like. Based upon this correlation, a prediction is made as to the TOC amount in the ultrapure water. Then, the predicted TOC value is indicated on the display 20A. At the same time, the estimated TOC value is compared with a predetermined value, for example, 5 ppb, by a comparator circuit or the like, with such an arrangement that, when the estimated TOC value rises above the reference value, the passage change-over valve 17 is switched so as to circulate the ultrapure water to the reflux pipe 18. When the estimated TOC value is lower than the reference value, the ultrapure water is caused to pass through the final filter 19, thereby removing from the water such impurities as fine particles The filtered water is then supplied to the use point 4. Fig. 7 shows an example of the correlation between the reduction $x$ in the resistivity value of ultrapure water and the total organic carbon content (TOC amount) in the same ultrapure water. This correlation shows that, under the constant irradiation of ultraviolet light, ultrapure water containing large quantities of organic matter therein exhibits $CO_2$ production which rises to a greater amount, thus reducing the resistivity value of ultrapure water extraordinarily. Whereas on the other hand, if small quantities of organic matter are present in ultrapure water, the amount of $CO_2$ produced is small, thus rendering the reduction of the resisitivity value smaller. The correlation may be expressed by, for example, the aforementioned equation (1) which was obtained experimentally, and TOC amount may be estimated by way of arithmetic processing. According to the correlation shown, when the resistivity difference $x$ is 10 MΩ -cm, the ultrapure water will contain 7 ppb of total organic carbon (TOC). Other graphs or computer tables may be prepared experimentally showing a particular relation between the resistivity difference and TOC amount. In this case, reference to the following table or computer table is made. Assuming that the resistivity is measured to read 5 MΩ -cm, then a resistivity difference is obtained by such subtraction as 18-5 = 13 (MΩ -cm), and reference to the table or computer table below as to this resistivity difference value shows that TOC = 20 ppb.

Table

| Resistivity Difference (MΩ -cm) | 13 | 12 | 11 | 10 | 9 | 8 | 7 | 6 |
|---|---|---|---|---|---|---|---|---|
| TOC (ppb) | 20 | 12 | 9 | 7 | 6 | 4.4 | 3.5 | 3 |

Using the resistivity measuring means 15, measurement is made continuously of the resistivity value of ultrapure water being flowed shortly after the exposure to ultraviolet light, then the reduction $x$ of the resistivity value is determined by the monitor means 20 comprising a known computer or the like. Thereafter a corresponding total organic matter content (TOC) amount in the ultrapure water is estimated optionally through either the calculation using the foregoing equation (1) or the computer table showing a correlation between the resistivity difference and TOC amount, which has been recorded beforehand. The TOC value thus obtained is then output to the display 20A or the like. Here, a temperature compensation may be made to correct any false resistivity values arising from resisitivty fluctuation in the temperature changes. The thermistor in the resistivity measuring means or the like may be used preferably as a means for making such correction. Further, preferably the total amount of organic matters (TOC) in ultrapure water is indicated as a value by ppb on the display 20A. Operators, in treating the ultrapure water manually or automatically using computers, must utilize the TOC data, which will be described below.

It is possible to precisely estimate or predict the amount of total organic matter (TOC) present even at extremely small quantities in the ultrapure water. The estimation per se is deemed so sufficient as to raise no practical trouble. In practice, therefore, such an arrangement may be provided that, if the resistivity reduction after the ultraviolet treatment is small, that is, the total organic matters amount in the ultrapure water is controlled below a predetermined value, then any anion and carbon dioxide gas in the water will be removed by the ion exchange means 16 so as to increase resistivity to a given value. Thereafter the ultrapure water flows toward the use point 4. If the resistivity reduction turns out to be greater, indicating the TOC amount to be in excess of the predetermined value, then the three-way valve 17 is switched to direct the ultrapure water to flow into the side of reflux pipe 18 for circulation therein, to thereby repeat the series of treatment steps as follows; the ion-exchange/organic-matter-removing treatment, the ultraviolet treatment and the ion exchange, until the TOC amount is retained at the predetermined value, at which time the three-way valve 17 is again switched to direct the water to flow to the use point. We, the inventors, conducted an experiment for obtaining results by practically actuating the above-constructed ultrapure water system 3, using a TOC measuring device, ANATEL A-100p, produced by ANATEL Co. and consequently, found that the results obtained are generally the same with those obtained in the previously discussed measurement processes based on the correlation between the resistivity difference and TOC amount.

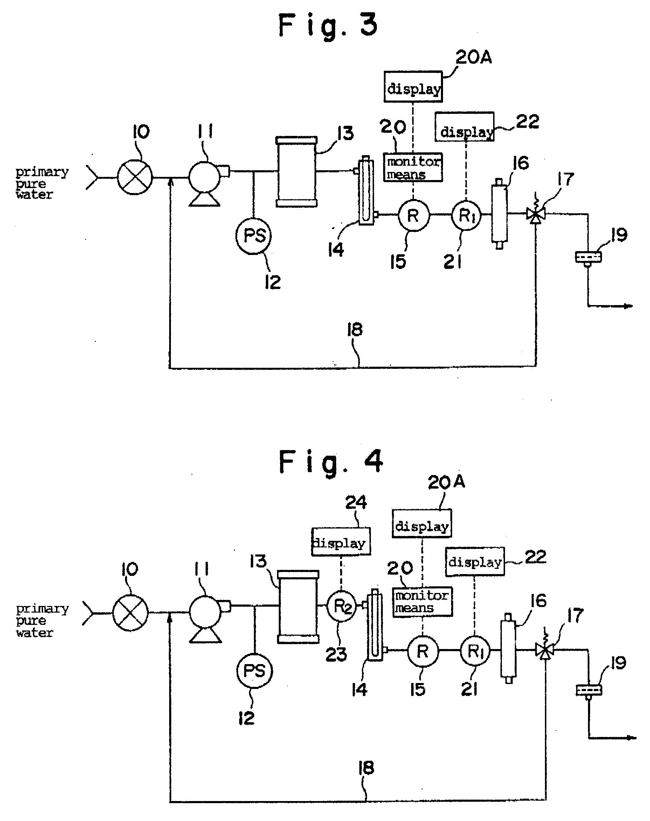

The above-mentioned embodiment is only a preferred embodiment of the present invention, and the present invention is not limited to this description. The present invention can be modified without departing from the scope of the present invention. For example, Fig. 3 shows another embodiment. In this embodiment, resistivity/conductivity measuring means 21 for quality control is disposed between ion exchange means 16 and a passage change-over valve 17. This arrangement serves to ascertain whether the resistivity value of the ultrapure water satisfies a predetermined resistivity value, for example, 18 MΩ - cm, or not. After removal of anion by the ion-exchange means 16, and only when such requirement is met, the water is allowed to be supplied to the use point. It is noted that the resistivity and conductivity meansuring means 21 does not contribute in measuring the TOC amount in the ultrapure water, but serves to switch the passage change-over valve 17, responsive to the resistivity of ultrapure water, which has been treated by the ion exchange means 16. The valve 17 is used to cause the water to flow into the reflux pipe 18 for circulation within the ultrapure water system, whenever the predetermined resistivity value is not satisfied. Further, by contrast, the means 21, upon sensing that the water satisfies the given resistivity value, will then switch the valve 17 to supply the water to the use point. The operation of the valve 17 may be done by an operator in response to a value reading in a display 22. Alternatively, the valve 17 may be operated under an automatic control using a output voltage from the resistivity/conductivity measuring means 21. In the latter case, for example, it may be so arranged that a suitable arithmetic processing device such as a computer or a comparator circuit is provided to effect a comparison between the measured value and a reference value (e.g. 18 MΩ -cm), with a program being set therein for automatic control such that if the measured value exceeds a given resistivity, the passage change-over valve 17 will be switched to effect flow communication with the use point 4. Whereas if the measured value is at or below the reference value, the valve 17 will remain in flow communication with the reflux pipe 18. In this connection, the measure value obtained by the resistivity/conductivity measuring means 21 is indicated on the display 22.

Fig. 4 shows a further embodiment. In this embodiment, a resistivity/conductivity measuring means 23 for quality control is interposed between the foregoing ion exchange means 13 and ultraviolet treatment means 14 shown in Fig. 3. With this arrangement, one can determine whether the ultrapure water that has been treated by the ion exchange satisfies the required resisitivity (18 MΩ -cm) or not. It is noted here that the resistivity/conductivity measuring means 23 does not directly contribute in measuring the TOC amount in the ultrapure water, but acts responsive to the ultrapure water, which has been treated by the ion exchange means 13, and which does not satisfy the required resistivity, and then in the sense that no precision exists in the TOC measurement, the means 23 serves to switch the valve 17 to effect flow to reflux pipe 18, thereby causing circulation of the water through the pipe 18. In the present embodiment, such an automatic control may be arranged, wherein a resisitivity or conductivity detected by the resistivity/conductivity measuring means 23 is input in the form of signal into a comparator circuit, for instance,, and compared with a reference value (18 MΩ -cm), and then, if the signal is determined to be greater than the reference value, the passage change-over valve 17 will be switched to effect flow communication with the use point 4, whereas if the signal is determined to be lower than the reference value, then the valve 17 will remain in flow communication with the reflux pipe 18.

INDUSTRIAL APPLICABILITY

Operation of the method of the present invention enables an ultrapure water having a low organic carbon or a low organic matter content to be economically obtained. The method can thus satisfy the requirements for improvement in quality of VLSI products and the requirements for an attempt to increase the analytical technical level by not only removing inorganic ions but also reducing the organic matter (carbon) content to the lowest limit, with an increase in resolution of analytical equipment and apparatus used in the fields of pharmaceutical and organic chemical reserach, bioengineering and other high-precision experiments and the like.

Claims

1. A method of measuring an amount of total organic matters content in an ultrapure water in an ultrapure water production system in which substantially a whole or said first ultrapure water being flowed is exposed to an ultraviolet light for decomposing the organic matters therein, to thereby produce a second ultrapure water having a low organic matters content, characterized in that an ultraviolet treatment is performed to said first ultrapure water which has undergone an ion-exchange/organic-matters-removing treatment, said first ultrapure water having a predetermined resistivity value or conductivity, then a measurement is continuously made to said first ultrapure water being introduced immediately after the exposure to said ultraviolet light, thereafter a measurement value obtained by said measurement is subtracted from a known predetermined resistivity value or conductivity to determine a resistivity difference, and from said resistivity difference, by referring to a correlation between a resistivity difference and an amount of carbon dioxide or substance that can be ionized, which is generated in ultrapure water, the amount of total organic matters content in said second ultrapure water is determined.

2. A method of measuring an amount or total organic matters in an ultrapure water in an ultrapure water production system according to Claim 1, wherein said ultraviolet treatment utilizes an ultraviolet light of approx. 185 nm wavelength.

3. An ultrapure water treatment system, which is provided in an ultrapure water production system for obtaining an ultrapure water having a predetermined resistivity value or conductivity from a primary pure water that has undergone a treatment by an ion exchange, reverse osmosis, distillation and so forth, characterized in that said ultrapure water treatment system comprises an ion-exchange/organic-matters-removing means, an ultraviolet treatment means for exposing to an ultraviolet light said ultrapure water being supplied from said ion-exchange/organic-matters-removing means, thereby decomposing organic matters therein, a means for measuring a resistivity value or conductivity of said ultrapure water being introduced immediately after the exposure to said ultraviolet light, an ion exchange means by which any of said resistivity value that is varied due to ions generated from said organic matters decomposed by said ultraviolet irradiation is brought back to a predetermied value or more, a valve able to change over a direction in which said ultrapure water is flowed, a reflux pipe which connects one of discharge ports of said valve with an upstream side of said ion-exchange/organic-matters-removing means to form a circulation passage, and a monitor means which determines a difference between said resistivity value or conductivity of said ultrapure water that has undergone said ultraviolet treatment and been measured by said resistivity value or conductivity measuring means and a known predetermined resistivity value or conductivity of said ultrapure water prior to said ultraviolet treatment, and determines the amount of total organic matters content in said ultrapure water on basis of a correlation between said resistivity or conductivity difference and an amount of carbon dioxide or substance that can be ionized in said ultrapure water.

4. A ultrapure water treatment system according to Claim 3, wherein a resistivity/condcutivity measuring means for quality control is disposed on a downstream side of said ion exchange means in order to detect whether or not said resistivity value of ultrapure water is recovered to said predetermined value.

5. A ultrapure water treatment system according to Claim 3 or 4, wherein a resistivity/conductivity measuring means for quality control is interposed between said ion-exchange/organic-matter-removingmeans and said ultraviolet treatment means in order to detect whether or not said resistivity value of ultrapure water is recovered to said predetermined value.

# Fig. 1

```
┌────────┐    ┌──────────────┐    ┌──────────────┐    ┌──────────────┐    ┌──────────────┐
│ raw    │    │       1      │    │      2       │    │      3       │    │      4       │
│ water  │ →  │ pretreatment │ →  │ primary ion- │ →  │ ultrapure    │ →  │              │
│        │    │ system       │    │ exchange     │    │ water        │    │ use point    │
│        │    │              │    │ treatment    │    │ treatment    │    │              │
└────────┘    └──────────────┘    └──────────────┘    └──────────────┘    └──────────────┘
```

# Fig. 2

EP 0 498 888 A1

Fig. 3

Fig. 4

14

# Fig. 5

34

31
30
32
33

$\overline{M}$        $\overline{M}$

# Fig. 6

34
31
30

Fig.7

$$Y=0.00458x^2+0.0012x+0.267$$

resistivity difference [MΩ·cm]

# INTERNATIONAL SEARCH REPORT

International Application No PCT/JP90/01434

**I. CLASSIFICATION OF SUBJECT MATTER** (if several classification symbols apply, indicate all) ⁴

According to International Patent Classification (IPC) or to both National Classification and IPC

Int. Cl⁵  G01N27/06

**II. FIELDS SEARCHED**

| Minimum Documentation Searched ⁷ | |
|---|---|
| Classification System | Classification Symbols |
| IPC | G01N27/06 |

Documentation Searched other than Minimum Documentation
to the Extent that such Documents are Included in the Fields Searched ⁸

| | |
|---|---|
| Jitsuyo Shinan Koho | 1926 – 1990 |
| Kokai Jitsuyo Shinan Koho | 1971 – 1990 |

**III. DOCUMENTS CONSIDERED TO BE RELEVANT ⁹**

| Category * | Citation of Document, ¹¹ with indication, where appropriate, of the relevant passages ¹² | Relevant to Claim No. ¹³ |
|---|---|---|
| Y | JP, B2, 63-46375 (Anatel Instrument Corp.), September 14, 1988 (14. 09. 88), DK, A0, 11985 & IL, A0, 73934 & EP, A2, 150923, JP, A2, 60-159642 & US, A, 4626413 & US, A, 4666860 & CA, A1, 1234183 & KR, B1, 8802324 & EP, B1, 150923 & AT, E, 43916 & US, A, 4868127 | 1-5 |
| A | US, A, 395894 (Shibron Corp.), May 25, 1976 (25. 05. 76), DE, A1, 2603752 & JP, B4, 58-33502 & GB, A, 1509391 & CA, A1, 1058907 & IT, A, 1053396 & DE, C2, 2603752 | 1-5 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 14, 1991 (14. 01. 91) | January 28, 1991 (28. 01. 91) |

| International Searching Authority | Signature of Authorized Officer |
|---|---|
| Japanese Patent Office | |